# EUROPEAN PATENT SPECIFICATION

(11) **EP 1 461 070 B1**
(45) Date of publication and mention of the grant of the patent: **24.01.2007**
(21) Application number: 02787467.6
(22) Date of filing: 20.12.2002
(51) Int. Cl.: A61K 38/26, A61K 31/35, A61P 3/10

(54) **COMBINED USE OF A GLP-1 COMPOUND AND AN ALDOSE REDUCTASE INHIBITOR**
KOMBINIERTE VERWENDUNG EINER GLP-1-VERBINDUNG UND EINES ALDOSE REDUKTASE INHIBITORS
UTILISATION COMBINEE D'UN COMPOSE GLP-1 ET D'UN INHIBITEUR D'ALDOSE REDUCTASE & xA;

(30) Priority: 29.12.2001 DK 200101969; 17.05.2002 DK 200200760
(43) Date of publication of application: 29.09.2004
(73) Proprietor: NOVO NORDISK A/S, 2880 Bagsvaerd (DK)
(72) Inventor: KNUDSEN, Lotte, Bjerre, DK-2500 Valby (DK); SELMER, Johan, DK-3520 Farum (DK)
(74) Representative: Stahr, Pia
(86) International application number: PCT/DK2002/000888
(87) International publication number: WO 2003/059372

(56) References cited:
- EP-A- 1 088 824
- EP-A- 1 092 711
- WO-A-01/66135
- US-A- 5 447 946
- DATABASE MEDLINE [Online] US NATIONAL LIBRARY OF MEDICINE (NLM), BETHESDA, MD, US; August 2001 (2001-08) TOFT-NIELSEN M B ET AL: "Determinants of the effectiveness of glucagon-like peptide-1 in type 2 diabetes." Database accession no. NLM11502823 XP002902901 & THE JOURNAL OF CLINICAL ENDOCRINOLOGY AND METABOLISM. UNITED STATES AUG 2001, vol. 86, no. 8, August 2001 (2001-08), pages 3853-3860, ISSN: 0021-972X

## Description

### FIELD OF THE INVENTION

The present invention relates to methods for treatment and/or prevention of diabetic late complications. More specifically, the methods and uses of the invention pertains to administration of a GLP-1 compound in combination with administration of a modulator of diabetic late complications.

### BACKGROUND OF THE INVENTION

Diabetes is a disorder of carbohydrate metabolism characterized by hyperglycemia and glucosuria resulting from insufficient production or utilization of insulin. Diabetes severely affects the quality of life of large parts of the populations in developed countries. Insufficient production of insulin is characterised as type 1 diabetes and insufficient utilization of insulin is type 2 diabetes.

Diabetics often tend to acquire a series of complications. Some of these complications exhibit fast onset once diabestes develops, whereas other types of complications are common only after years of diabetes. The latter complications are therefore often termed "diabetic late complications", and they typically comprise nephropathy, retinopathy, neuropathy and hypertension.
Nephropathy is a diabetic kidney disease, the initiation of which is closely related to the degree and duration of metabolic disturbances, as reflected predominantly by hyperglycaemia. Incipient nephropathy or microalbuminuria may over time progress to overt nephropathy characterized in macroalbuminuria. Diabetic nephropathy is often treated with inhibitors of advanced glycation, aldose reductase and protein kinase C, hereby attenuating or reversing the disease state.
Diabetic retinopathy is the major preventable cause of visual loss in adults. Good control of diabetes descreases the risk of diabetic retinopathy. A large number of patients need laser photocoagulation and some require vitreoretinal surgery to decrease visual loss. Diagnosis in time for intervention remains pivotal as diabetic retinopathy is symptomless until blurred vision or sudden visual loss. Thus, screening of patients with diabetes is crucial for timely diagnosis. Hence, not only are therapeutic approaches to visual recovery important but perhaps even more important are therapeutic approaches to prevention of the progression of diabetic retinopathy.
Neuropathy is another diabetic late complication which is common among diabetics since approximately 50% of diabetics develops diabetic neuropathy. Since diabetes can affect several different components of the peripheral nervous system, diabetic neuropathy is not a single disorder but rather a series of distinct clinical syndromes. The most common type is distal symmetrical sensorimotor polyneuropahty (DPN) which exhibits progressive loss of sensation and less frequently motor function. Diabetes may also damage the autonomic nervous system, resulting in diabetic autonomic neuropathy (DAN). Neuropathy often includes recurrent infections, non-healing ulcers and amputation of toes and feet. Therapeutic options are mainly pain relief and are directed to treat the symptoms. Thus, diabetic neuropathy is one of the most morbid and costly complications of diabetes.
Hypertension is also a very common diabetic late complication, since it affects 40-60% of type 2 diabetics between the ages of 40-75. Hypertension is often unrecognized and undertreated, in spite of the fact that if hypertension is controlled this reduces diabetes related deaths, stroke, macrovascular disease, microvascular disease and heart failure. The drugs used for treatment of hypertension are mainly angiotension converting enzyme inhibitors, angiotensin II receptor antagonist and β-blockers.

Taken together the common diabetic late complications significantly impair the quality of life of diabetics and also represents a large burden on the health care system. Hence, to relieve this burden accompanying diabetes new therapeutic approaches are needed. The therapeutic benefits from combined use of drugs is one of the solutions whereby better disease control and delayed disease onset can be attained.

Human GLP-1 is a 37 amino acid residue peptide originating from preproglucagon which is synthesized *i.a.* in the L-cells in the distal ileum, in the pancreas and in the brain. GLP-1 is an important gut hormone with regulatory function in glucose metabolism and gastrointestinal secretion and metabolism. Processing of preproglucagon to give GLP-1 (7-36)amide, GLP-1 (7-37) and GLP-2 occurs mainly in the L-cells. A simple system is used to describe fragments and analogues of this peptide. Thus, for example, Gly⁸-GLP-1 (7-37) designates a fragment of GLP-1 formally derived from GLP-1 by deleting the amino acid residues Nos. 1 to 6 and substituting the naturally occurring amino acid residue in position 8 (Ala) by Gly. Similarly, Lys³⁴(N^{a}-tetradecanoyl)-GLP-1 (7-37) designates GLP-1 (7-37) wherein the ε-amino group of the Lys residue in position 34 has been tetradecanoylated. PCT publications WO 98/08871 and WO 99/43706 disclose derivatives of GLP-1 analogs, which have a lipophilic substituent. These stable derivatives of GLP-1 analogs have a protracted profile of action compared to the corresponding GLP-1 analogs.
A number of structural analogs of GLP-1 were isolated from the venom of the Gila monster lizards *(Heloderma suspectum* and *Heloderma horridum).* Exendin-4 is a 39 amino acid residue peptide isolated from the venom of Heloderma horridum, and this peptide shares 52% homology with GLP-1. Exendin-4 is a potent GLP-1 receptor agonist which has been shown to stimulate insulin release and ensuing lowering of the blood glucose level when injected Into dogs. Exendin-4, exendin-4 analogs and derivatives of any of these as well as methods for productionn thereof can be found in WO 99/43708. WO 00/66629, and WO 01/04156 The group of GLP-1(1-37), exendin-4(1-39), analogs thereof and derivatives thereof, (hereinafter designated GLP-1 compounds) are potent insulinotropic agents.

### SUMMARY OF THE INVENTION

In accordance with the present invention, a pharmaceutical combination is provided for use in treatment of diabetic neuropathy which combination comprises a GLP-1 compound and an aldose reductase inhibitor.

One object of the present invention is to provide methods, which can effectively be used in the treatment or prophylaxis of diabetic neuropathy.

The invention includes a method for the treatment or prophylaxis of diabetic neuropathy which method comprises administration of a GLP-1 compound and an aldose reductase inhibitor to a patient in need thereof.

In one embodiment of the invention, the GLP-1 compound is a stable derivative of a GLP-1 analog. A preferred embodiment is a GLP-1 analog with a lipophilic substituent, preferably Arg³⁴. Lys²⁰(N⁶-(γ-Glu(N^{α}-hexadecanoyl)))-GLP-1(7-3).

In yet another embodiment of the invention the aldose reductase inhibitor and the GLP-1 compound are administered in suboptimal dosages. In yet another embodiment of the invention the aldose reductase inhibitor and the GLP-1 compound are administered in amounts and for a sufficient time to produce a synergistic effect.

### DEFINITIONS

Co-administration : In the context of the present application, co-administration of two compounds is defined as administration of the two compounds to the patient within 24 hours, including separate administration of two medicaments each containing one of the compounds as well as simultaneous administration whether or not the two compounds are combined in one formulation or whether they are in two separate formulations.

Effective dosage: An effective dosage is a dosage which is sufficient in order for the treatment of the patient to be effective.

GLP-1 compound: A GLP-1 analog or a derivative thereof or exendin-4, an exendin-4 analog or a derivative thereof. For example, Gly⁸-GLP-1 (7-37) designates a fragment of GLP-1 formally derived from GLP-1 by deleting the amino acid residues Nos. 1 to 6 and substituting the naturally occurring amino acid residue in position 8 (Ala) by Gly. Examples of derivatives of GLP-1 analogs can be found in WO 98/08871 and WO 99/43706. Likewise, exendin-4 and analogs and derivatives thereof are described in WO 99/43708, WO 00/66629 and WO 01/04146. In the present application an analog of a parent protein means a protein wherein one or more amino acid residues of the parent protein have been substituted by other amino acid residues and/or wherein one or more amino acid residues of the parent protein have been deleted and/or wherein one or more amino acid residues have been inserted into the parent protein. Such an insertion of amino acid residues can take place at the C-terminal, at the N-terminal, within the peptide sequence or a combination thereof. In the present application the term "derivative" means a chemical derivative of the parent protein, i.e. a protein wherein at least one of the constituent amino acid residues are covalently modified so that it is no longer a genetically encoded amino acid. Examples of such covalent modifications are acylations, alkylations, esterifications, glycosylations and PEGylations.

Medicament : Pharmaceutical composition suitable for administration of the pharmaceutically active compound to a patient.

Suboptimal dosage: A suboptimal dosage of a pharmaceutically active compound is a dosage which is below the optimal dosage for that compound when used in single-compound therapy.

Synergistic effect : A synergistic effect of two compounds is in terms of statistical analysis an effect which is greater than the additive effect which results from the sum of the effects of the two individual compounds.

Treatment : In this application treatment is defined as the management and care of a patient for the purpose of combating the disease, condition, or disorder and includes the administration of the active compounds to prevent the onset of the symptoms or complications, or alleviating the symptoms or complications, or eliminating the disease, condition, or disorder.

Stable derivative of a GLP-1 analog: A GLP-1 analog or a derivative thereof which exhibits an in vivo plasma elimination half-life of at least 10 hours in man, as determined by the method described below. Examples of stable derivatives of GLP-1 analogs can be found in WO 98/08871 and WO 99/43706. The method for determination of plasma elimination half-life of a compound in man is : The compound is dissolved in an isotonic buffer, pH 7.4, PBS or any other suitable buffer. The dose is injected peripherally, preferably in the abdominal or upper thigh. Blood samples for determination of active compound are taken at frequent intervals, and for a sufficient duration to cover the terminal elimination part (e.g. Pre-dose, 1, 2, 3, 4, 5, 6, 7, 8, 10, 12, 24 (day 2), 36 (day 2), 48 (day 3), 60 (day 3), 72 (day 4) and 84 (day 4) hours post dose). Determination of the concentration of active compound is performed as described in Wilken et al., Diabetologia 43(51):A143, 2000. Derived pharmacokinetic parameteres are calculated from the concentration-time data for each individual subject by use of non-compartmental methods, using the commercially available software WinNonlin Version 2.1 (Pharsight, Cary, NC, USA). The terminal elimination rate constant is estimated by log-linear regression on the terminal log-linear part of the concentration-time curve, and used for calculating the elimination half-life.

### DETAILED DESCRIPTION OF THE INVENTION

It is believed that in the treatment of diabetic late complications the combined treatment with a GLP-1 compound and an aldose reductase inhibitor provides a favourable effect with a minimum of side effects as compared to single compound therapy.

A synergistic effect of two compounds permits the dosages of these compounds In the combined treatment to be below the optimal dosages of the individual compounds in single-compound treatment Thus, these suboptimal dosages of the individual compounds reduce side effects since lower dosages are needed for the same therapeutic effect in the combined treatment.

Accordingly, the present invention relates to methods for treatment of diabetic neuropathy which method comprises administration of a GLP-1 compound and an aldose reductase inhibitor to a patient in need thereof.

The methods comprise administration of an effective amount of a GLP-1 compound and administration of an effective amount of an aldose reductase inhibitor. The two compounds may be co-adminlstered or they may be administered separately as two medicaments. Furthermore, the first compound may be administered in a regimen, which additionally comprises treatment with the second compound. Hence, according to the present invention the only provision is that there must be overlapping periods of treatment with the GLP-1 compound and the aldose reductase inhibitor.

In one embodiment of the invention, the GLP-1 compound is a stable derivative of a GLP-1 analog. A preferred embodiment Is a GLP-1 analog with a lipophilic substituent, preferably Arg³⁴, Lys²⁸(N'-(γ-Glu(N^{α}-hexadecanoyl)))-GLP-1(7-37). Arg³⁴, Lys²⁸(N^{k}-(γ-Glu(N^{α}-hexadecanoyl)))-GLP-1(7-37) is disclosed in WO 98/08871. In another embodiment of the invention, the GLP-1 compound is exendin-4 or an analog or derivative thereof.

In another embodiment of the invention, the GLP-1 compound is an analog of GLP-1 (7-37) which has less than 10 amino acid residues different from those in GLP-1 (7-37), less than 5 amino acid residues different from those in GLP-1 (7-37), less than 3 amino acid residues different from those in GLP-1 (7-37), preferably only one amino acid residue different from that in GLP-1 (7-37).
In another embodiment of the invention, the GLP-1 compound is selected from the group consisting of Gly⁸-GLP-1 (7-36)-amide, Gly⁸-GLP-1 (7-37), Val⁸-GLP-1 (7-36)-amide, Val⁸-GLP-1 (7-37), Val⁸Asp²²-GLP-1(7-36)-amide, Val⁸Asp²²-GLP-1(7-37), Val⁸Glu²²-GLP-1(7-36)-amide , Val⁸Glu²²-GLP-1 (7-37), Val⁸Lys²²-GLP-1(7-36)-amide, Val⁸Lys²²-GLP-1(7-37), Val⁸Arg²²-GLP-1(7-36)-amide, Val⁸Arg²²-GLP-1 (7-37), Val⁸His²²-GLP-1 (7-36)-amide, Val⁸His²²-GLP-1 (7-37), Arg²⁶-GLP-1 (7-37); Arg³⁴-GLP-1 (7-37); Lys³⁶-GLP-1 (7-37); Arg^{26,34}Lys³⁶-GLP-1 (7-37); Arg^{26,34}-GLP-1 (7-37); Arg^{26,34}Lys⁴⁰-GLP-1 (7-37); Arg²⁶Lys³⁶-GLP-1 (7-37); Arg³⁴Lys³⁶-GLP-1 (7-37); Val⁸Arg²²-GLP-1(7-37); Met⁸Arg²²-GLP-1 (7-37);Gly⁸His²²-GLP-1 (7-37); Val⁸His²²-GLP-1(7-37); Met⁸His²²-GLP-1 (7-37);His³⁷-GLP-1 (7-37); Gly⁸-GLP-1 (7-37); Val⁸-GLP-1 (7-37); Met⁸-GLP-1 (7-37);Gly⁸Asp²²-GLP-1 (7-37); Val⁸ASp²²-GLP-1 (7-37); Met⁸Asp²²-GLP-1(7-37); Gly⁸Glu²²-GLP-1(7-37); Val⁸Glu²²-GLP-1(7-37); Met⁸Glu²²-GLP-1 (7-37); Gly⁸Lys²²-GLP-1(7-37); Val⁸Lys²²-GLP-1 (7-37); Met⁸Lys²²-GLP-1(7-37); Gly⁸Arg²²-GLP-1 (7-37); Val⁸Lys²²His³⁷-GLP-1(7-37); Gly⁸Glu²²His³⁷-GLP-1(7-37); Val⁸Glu²²His³⁷-GLP-1 (7-37); Met⁸Glu²²His³⁷-GLP-1(7-37); Gly⁸Lys²²His³⁷-GLP-1(7-37); Met⁸Lys²²His³⁷-GLP-1 (7-37); Gly⁸Arg²²His³⁷-GLP-1(7-37); Val⁸Arg²²His³⁷-GLP-1(7-37); Met⁸Arg²²His³⁷-GLP-1(7-37); Gly⁸His²²His³⁷-GLP-1 (7-37); Val⁸His²²His³⁷-GLP-1(7-37); Met⁸His²²His³⁷-GLP-1 (7-37); Gly⁸His³⁷-GLP-1 (7-37); Val⁸His³⁷-GLP-1(7-37); Met⁸His³⁷-GLP-1(7-37); Gly⁸Asp²² His³⁷-GLP-1 (7-37); Val⁸Asp²²His³⁷-GLP-1 (7-37); Met⁸Asp²²His³⁷-GLP-1 (7-37); Arg²⁶-GLP-1 (7-36)-amide; Arg³⁴-GLP-1 (7-36)-amide; Lys³⁶-GLP-1 (7-36)-amide; Arg^{26,34}Lys³⁶-GLP-1 (7-36)-amide; Arg^{26,34}-GLP-1 (7-36)-amide; Arg^{26,34}Lys⁴⁰-GLP-1 (7-36)-amide; Arg²⁶Lys³⁶-GLP-1 (7-36)-amide; Arg³⁴Lys³⁶-GLP-1(7-36)-amide; Gly⁸-GLP-1(7-36)-amide; Val⁸-GLP-1 (7-36)-amide; Met⁸-GLP-1 (7-36)-amide;Gly⁸Asp²²-GLP-1 (7-36)-amide; Gly⁸Glu²²His³⁷-GLP-1 (7-36)-amide; Val⁸Asp²²-GLP-1(7-36)-amide; Met⁸Asp²²-GLP-1 (7-36)-amide;Gly⁸Glu²²-GLP-1 (7-36)-amide; Val⁸Glu²²-GLP-1 (7-36)-amide; Met⁸Glu²²-GLP-1(7-36)-amide; Gly⁸Lys²²-GLP-1 (7-36)-amide; Val⁸Lys²²-GLP-1(7-36)-amide; Met⁸Lys²²-GLP-1(7-36)-amide; Gly⁸His²²His³⁷-GLP-1 (7-36)-amide; Gly⁸Arg²²-GLP-1(7-36)-amide; Val⁸Arg²²-GLP-1(7-36)-amide; Met⁸Arg²²-GLP-1(7-36)-amide; Gly²²His²²-GLP-1 (7-36)-amide; Val⁸His²²-GLP-1 (7-36)-amide; Met⁸His²²-GLP-1 (7-36)-amide;His³⁷-GLP-1 (7-36)-amide; Val⁸Arg²²His³⁷-GLP-1 (7-36)-amide; Met⁸Arg²²His³⁷-GLP-1 (7-36)-amide; Gly⁸His³⁷-GLP-1(7-36)-amide; Val⁸His³⁷-GLP-1 (7-36)-amide; Met⁸His³⁷-GLP-1(7-36)-amide;Gly⁸Asp²² His³⁷-GLP-1 (7-36)-amide; Val⁸Asp²²His³⁷-GLP-1 (7-36)-amide; Met⁸Asp²²His³⁷-GLP-1(7-36)-amide; Val⁸Glu²²His³⁷-GLP-1 (7-36)-amide; Met⁸Glu²²His³⁷-GLP-1(7-36)-amide; Gly⁸Lys²²His³⁷-GLP-1 (7-36)-amide; Val⁸Lys²²His³⁷-GLP-1 (7-36)-amide; Met⁸Lys²²His³⁷-GLP-1(7-36)-amide;Gly⁸Arg²²His³⁷-GLP-1(7-36)-amide; Val⁸His³⁷-GLP-1(7-36)-amide; Met⁸His²²HiS³⁷-GLP-1(7-36)-amide; and derivatives thereof.

In a preferred embodiment the aldose reductase inhibitor is fidarest

In yet another embodiment the GLP-1 compound and the aldose reductase inhibitor co-administered to the patient. The two compounds may be administered as separately formulated compounds or they may be administered as one formulation comprising both compounds. In a further embodiment, the GLP-1 compound is administered in a regimen, which additionally comprises administration of the aldose reductase inhibitor In a preferred embodiment the GLP-1 compound is a parenteral medicament. In another preferred embodiment the aldose reductase inhibitor is an oral medicament.

In yet another embodiment, the GLP-1 compound and the aldose reductase inhibitor are administered in suboptimal dosages, i.e. dosages lower than the optimal dosages for single compound therapy.

In yet another embodiment, the dosage of said GLP-1 compound is from 0.5 µg/kg/day to 10 µg/kg/day.

In yet another embodiment, the dosage of said GLP-1 compound is from 0.1 µg/kg/day to 1 µg/kg/day.

In a further embodiment the GLP-1 compound and the aldose reductase inhibitor are administered in sufficient amount and for a sufficient time to produce a synergistic effect, preferably for at least 4 weeks.

The subject or patient is preferably a mammal, more preferably a human.

The route of administration may be any route, which effectively transports the active compound to the appropriate or desired site of action, such as oral, nasal, buccal, pulmonal, transdermal or parenteral.

Pharmaceutical compositions (or medicaments) containing a GLP-1 compound, such as Arg³⁴, Lys²⁶(N^{ε}-(γ-Glu(N^{α}-hexadecanoyl)))-GLP-1(7-37), may be administered parenterally to patients in need of such a treatment Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of a GLP-1 compound in the form of a nasal or pulmonal spray. As a still further option, the GLP-1 compound can also be administered transdermally, e.g. from a patch, optionally a iontophoretic patch, or transmucosally, *e.g*. bucally. The above-mentioned possible ways to administer stable derivatives of GLP-1 analogs are not considered as limiting the scope of the invention.

Pharmaceutical compositions containing GLP-1 compounds, such as Arg³⁴, Lys²⁶(N^{c}-(γ-Glu(N^{α}-hexadecanoyl)))-GLP-1 (7-37), may be prepared by conventional techniques, *e.g.* as described in Remington's *Pharmaceutical Sciences,* 1985 or in Remington: *The Science and Practice of Pharmacy,* 19^{th} edition, 1995.

Thus, the injectable compositions of GLP-1 compounds can be prepared using the conventional techniques of the pharmaceutical industry which involves dissolving and mixing the ingredients as appropriate to give the desired end product

According to one procedure, e.g. Arg³⁴, Lys²⁶(N^{c}-(γ-Glu(N^{α}-hexadecanoyl)))-GLP-1(7-37) is dissolved in an amount of water which is somewhat less than the final volume of the composition to be prepared. An isotonic agent, a preservative and a buffer are added as required and the pH value of the solution is adjusted - if necessary - using an add, e.g. hydrochloric acid, or a base, e.g. aqueous sodium hydroxide as needed. Finally, the volume of the solution is adjusted with water to give the desired concentration of the ingredients.

Examples of isotonic agents are sodium chloride, mannitol and glycerol.

Examples of preservatives are phenol, m-cresol, methyl p-hydroxybenzoate and benzyl alcohol.

Examples of suitable buffers are sodium acetate and sodium phosphate.

Further to the above-mentioned components, solutions containing a GLP-1 compound may also contain a surfactant In order to improve the solubility and/or the stability of the peptide.

According to one embodiment of the present invention, the GLP-1 compound is provided in the form of a composition suitable for administration by injection. Such a composition can either be an injectable solution ready for use or it can be an amount of a solid composition, *e.g.* a lyophilised product, which has to be dissolved in a solvent before it can be injected. The injectable solution preferably contains not less than about 0.1 mg/ml, typically from 0.1 mg/ml to 10 mglml, such as from 1 mg/ml to 5 mg/ml of GLP-1 compound.

GLP-1 compounds such as Arg³⁴, Lys²⁰(N^{ε}-(γ-Glu(N^{α}-hexadecanoyl)))GLP-1(7-37) can be used in the treatment of various diseases. The optimal dose level for any patient (effective amount) will depend on the disease to be treated and on a variety of factors including the efficacy of the specific GLP-1 compound employed, the age, body weight, physical activity, and diet of the patient, on which other drugs GLP-1 is combined with, and on the severity of the case.

Pharmaceutical compositions (or medicaments) containing an aldose reductase inhibitor may be administered by suitable dosage forms such as oral, nasal, pulmonal, buccal or transdermal to patients in need of such a treatment. The preferred route of administration aldose reductase inhibitor is orally. Pharmaceutical compositions containing an aldose reductase inhibitor may be prepared by conventional techniques, *e.g*. as described in Remington: The Science and Practice of Pharmacy, 19th Edition, Gennaro, Ed., Mack Publishing Co., Easton, PA, 1995.

Typical compositions of an aldose reductase inhibitor, include a crystalline compound associated with a pharmaceutically acceptable excipient, which may be a carrier or a diluent or be diluted by a carrier, or endosed within a carrier, which can be in the form of a capsule, sachet, paper or other container. In making the compositions, conventional techniques for the preparation of pharmaceutical compositions may be used. For example, the active compound will usually be mixed with a carrier, or diluted by a carrier, or enclosed within a carrier, which may be in the form of a ampoule, capsule, sachet, paper, or other container. When the carrier serves as a diluent, it may be solid, semi-solid, or liquid material, which acts as a vehicle, excipient, or medium for the active compound. The active compound can be adsorbed on a granular solid container for example in a sachet. Some examples of suitable carriers are water, salt solutions, alcohol's, polyethylene glycol's, polyhydroxyethoxylated castor oil, peanut oil, olive oil, gelatine, lactose, terra alba, sucrose, cyclodextrin, amylose, magnesium stearate, talc, gelatine, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art.

The pharmaceutical compositions can be sterilized and mixed, if desired, with auxiliary agents, emulsifiers, salt for influencing osmotic pressure, buffers and/or colouring substances and the like, which do not deleteriously react with the active compound.

If a solid carrier is used for oral administration, the preparation may be tabletted, placed in a hard gelatine capsule in powder or pellet form or it can be in the form of a troche or lozenge. If a liquid carrier is used, the preparation may be in the form of a syrup, emulsion, soft gelatine capsule or sterile injectable liquid such as an aqueous or non-aqueous liquid suspension or solution.

For nasal administration, the preparation may contain the compound of the present invention dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, cornstarch, and/or potato starch. A syrup or elixir can be used in cases where a sweetened vehide can be employed.

A typical tablet of an aldose reductase inhibitor which may be prepared by conventional tabletting techniques, may contain:

| Core: | |
|---|---|
| Active compound | 5 mg |
| Colloidal silicon dioxide (Aerosil) | 1.5 mg |
| Cellulose, microcryst. (Avicel) | 70 mg |
| Modified cellulose gum (Ac-Di-Sol) | 7.5 mg |
| Magnesium stearate. | Ad. |

| Coating: | |
|---|---|
| HPMC approx | 9 mg |
| *Mywacett 9-40 T approx. | 0.9 mg |

| | |
|---|---|
| *Acylated monoglyceride used as plasticizer for film coating. | |

For example, in the treatment of adult humans, dosages from 0.01 mg/day to 10 mg/day, preferably from 0.1 mg/day to 3 mg/day may be used. A most preferable dosage is less-than 2 mg/day. In choosing a regimen for patients it may frequently be necessary to begin with a dosage of from about 2 to about 10 mg per day and when the condition is under control to reduce the dosage as low as from about 0.01 to about 3 mg per day. The exact dosage will depend upon the mode of administration, on the therapy desired, the administration form, the subject to be treated and the body weight of the subject to be treated.

Generally, the aldose reductase inhibitor of the present invention are dispensed in unit dosage form comprising from about 0.01 to about 10 mg of active ingredient together with a pharmaceutically acceptable carrier per unit dosage.

Usually, dosage forms suitable for oral, nasal, pulmonary or transdermal administration comprise from about 0.01 mg to about 10 mg, preferably from about 0.1 mg to about 3 mg of the compound of the invention admixed with a pharmaceutically acceptable carrier or diluent.

Irrespective of the dosage forms for the GLP-1 compound and for the aldose reductase inhibitor, they may advantageously be supplied as a kit for treatment of diabetic neuropathy. The kit may contain a single dosage form or it may contain two dosage forms, i.e. one for each compound to be administered.

In one embodiment the dosage of said GLP-1 compound is from 0.5 µg/kg/day to 10 µg/kg/day, and the dosage of said aldose reductase inhibitor from 0.01 mg/day to 10 mg/day. In another embodiment the dosage of said GLP-1 compound is from 0.1 µg/kg/day to 1 µg/kg/day, and the dosage of said aldose reductase inhibitor is from 0.01 mg/day to 10 mg/day. In another embodiment the dosage of said GLP-1 compound is from 0.5 µg/kg/day to 10 µg/kg/day, and the dosage of said aldose reductase inhibitor is from 0.1 mg/day to 3 mg/day In another embodiment the dosage of said GLP-1 compound is from 0.1 µg/kg/day to 1 µg/kg/day, and the dosage of said aldose reductase inhibitor is from 0.1 mg/day to 3 mg/day. In another embodiment the dosage of said GLP-1 compound is from 0.1 µg/kg/day to 1 µg/kg/day, and the dosage of said aldose reductase inhibitor is from 0.2 mg/day to 2 mg/day.

The combined treatment with a GLP-1 compound and an aldose reductase inhibitor may also be combined with a third or more further pharmacologically active substances, e.g. selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Examples of these pharmacologically active substances are: Insulin, GLP-1 agonists, sulphonylureas, biguanides, meglitinides, glucosidase inhibitors, glucagon antagonists, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidernic agents as HMG CoA inhibitors (statins), compounds lowering food intake, RXR agonists and agents acting on the ATP-dependent potassium channel of the β-cells, Cholestyramine, colestipol, clofibrate, gemfibrozil, lovastatin, pravastatin, simvastatin, probucol, dextrothyroxine, neteglinide, repaglinide; β-blockers such as alprenolol, atenolol, timolol, pindolol, propranolol and metoprolol, ACE (angiotensin converting enzyme) inhibitors such as alatriopril, benazepril, captopril, enalapril, fosinopril, lisinopril, quinapril and ramipril, calcium channel blockers such as nifedlpine, felodipine, nicardipine, isradipine, nimodipine, diltiazem and verapamil, and α-blockers such as doxazosin, urapidil, prazosin and terazosin; CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocytestimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators, TR β agonists; histamine H3 antagonists.

It should be understood that any suitable combination of the compounds according to the invention with one or more of the above-mentioned compounds and optionally one or more further pharmacologically active substances are considered to be within the scope of the present invention.

### EXAMPLES

### Test of efficacy of combined use of GLP-1 and a modulator of diabetic late complications

### Patients

Type 2 diabetic patients with no diagnosed diabetic late complications (DLC) or a group of type 2 diabetic patients with already diagnosed microalbuminuria. Subjects are allocated to groups to match body weight and HbA_{1c}. All patients discontinue any antidiabetic medication at least two weeks before start of study.

### Dosing groups

1) Control, dosed with both vehicles
2) GLP-1 (or Arg³⁴, Lys²⁶(N^{ε}-(γ-Glu(N^{α}-hexadecanoyl)))-GLP-1(7-37)) + vehicle
3) Vehicle + modulator of DLC
4) GLP-1 (or Arg³⁴, Lys²⁶(N^{ε}-(γ-Glu(N^{α}-hexadecanoyl)))-GLP-1(7-37)) + modulator of DLC Modulators of DLC:
Aldose reductase inhibitor: Fidarest
Protein Kinase C inhibitor: LY333531 (ruboxistaurin)
ACE inhibitor: Captopril, alatriopril, enalapril, fosinopril, lisinopril, quinapril, ramipril, spirapril, benazepril, imidapril, trandolapril, or perindopril erbumine
Angiotensin II receptor antagonist: Losartan, valsartan, or irbesartan.
Non specific β-adrenergic antagonist: propranolol, nadolol, timolol or pindolol
β₁-adrenergic antagonist: metoprol, atenolol, esmolol or acebutolol

Patients are dosed for a period from 3 months up to several years depending on what DLC is to be studied (dosing according to kinetics of chosen compounds). Doses of both GLP-1 and modulator of DLC are chosen slightly lower than what would be needed for optimal treatment of DLC with mono-therapy to enable detection of synergistic or additive effect in group 4.

### Experimental set-up

At the start and at the end of the dosing period blood samples are obtained from fasted subjects and plasma levels of LDL, HDL, VLDL, triglycerides, FFA, glucose, HbA_{1c}, C-peptide and glucagon are measured.
Furthermore, evaluation of DLC is done according to one or more of the following methods:

### Nephropathy:

Urinary albumin excretion rates (UAE): Increased UAE is a very early marker of diabetic kidney disease.

Transcapillary escape rate for albumin (TERalb): A measure of generalised increased leakage from the vasculature is the increased permeability for albumin. This is measured as the disappearance of I¹²⁵-labelled albumin from the blood stream. TERalb is positively correlated to UAE.

### Neuropathy:

Nerve conduction velocity (NCV): NCV is a measure of peripheral motor nerve (n.isciadicus) function.

### Retinopathy:

Opthalmoscopic examination: Microaneurysms can be detected by direct opthalmoscopy or with the use of fluorescine angiography

### Hypertension:

Blood pressure measurements: Blood pressure is recorded over 24 hours at various time points during the dosing period.

### Evaluation of results

Synergistic effect of GLP-1 and DLC modulator treatment is shown by functional and morphological markers of LDC being less developed in group 4 compared to the sum of effects in group 2 or 3. Additive effect of GLP-1 and DLC modulator treatment is shown by functional and morphological markers of LDC being less developed in group 4 than in group 2 or 3.

## Claims

1. Use of a GLP-1 compound and an aldose reductase inhibitor for the preparation of one or more medicaments for the treatment of diabetic neuropathy in a patient in need thereof.

2. Use according to claim 1, wherein the GLP-1 compound is a stable derivative of a GLP-1 analog.

3. Use according to any one of claims 1-2, wherein the GLP-1 compound is Arg³⁴, Lys²⁶(N^{ε}-(γ-Glu(N^{α}-hexadecanoyl)))-GLP-1(7-37).

4. Use according to claim 1, wherein the GLP-1 compound is exendin-4 or an analog or derivative thereof.

5. Use according to claim 1, wherein the aldose reductase inhibitor is fidarest.

6. Use according to any one of claims 1-5, wherein the GLP-1 compound is administered in a regimen which additionally comprises administration of an aldose reductase inhibitor.

7. Use according to any one of claims 1-6, wherein the GLP-1 compound and the aldose reductase inhibitor are co-administered.

8. Use according to any one of claims 1-7, wherein the GLP-1 compound is a parenteral medicament.

9. Use according to any one of claims 1-8, wherein the aldose reductase inhibitor is an oral medicament.

10. Use according to any one of claims 1-9, wherein the GLP-1 compound and the aldose reductase inhibitor are administrered in suboptimal dosages.

11. Use according to any one of claims 1-10, wherein the dosage of GLP-1 compound is from 0.5 µg/kg/day to 10 µg/kg/day.

12. Use according to any one of claims 1-11, wherein the dosage of GLP-1 compound is from 0.1 µg/kg/day to 1 µg/kg/day.

13. Use according to any one of claims 1-12, wherein the dosage of the aldose reductase inhibitor is from 0.01 mg/day to 10 mg/day, preferably from 0.1 mg/day to 3 mg/day, most preferable less than 2 mg/day.

## Patentansprüche

1. Verwendung einer GLP-1-Verbindung und eines Aldosereduktasehemmers zur Herstellung von einem oder mehreren Medikamenten zur Behandlung von Diabetesneuropathie bei einem dies benötigenden Patienten.

2. Verwendung nach Anspruch 1, wobei die GLP-1-Verbindung ein stabiles Derivat eines GLP-1-Analogons ist.

3. Verwendung nach einem der Ansprüche 1-2, wobei die GLP-1-Verbindung Arg³⁴, Lys²⁶(N^{ε}-(γ-Glu(N^{α}-hexadecanoyl)))-GLP-1(7-37) ist.

4. Verwendung nach Anspruch 1, wobei die GLP-1-Verbindung Exendin-4 oder ein Analogon oder Derivat davon ist.

5. Verwendung nach Anspruch 1, wobei der Aldosereduktasehemmer Fidarest ist.

6. Verwendung nach einem der Ansprüche 1-5, wobei die GLP-1-Verbindung in einer Kur verabreicht wird, die zusätzlich die Verabreichung eines Aldosereduktasehemmers umfasst.

7. Verwendung nach einem der Ansprüche 1-6, wobei die GLP-1-Verbindung und der Aldosereduktasehemmer gemeinsam verabreicht werden.

8. Verwendung nach einem der Ansprüche 1-7, wobei die GLP-1-Verbindung ein parenterales Medikament ist.

9. Verwendung nach einem der Ansprüche 1-8, wobei der Aldosereduktasehemmer ein orales Medikament ist.

10. Verwendung nach einem der Ansprüche 1-9, wobei die GLP-1-Verbindung und der Aldosereduktasehemmer in suboptimalen Dosierungen verabreicht werden.

11. Verwendung nach einem der Ansprüche 1-10, wobei die Dosierung der GLP-1-Verbindung 0,5 µg/kg/Tag bis 10 µg/kg/Tag beträgt.

12. Verwendung nach einem der Ansprüche 1-11, wobei die Dosierung der GLP-1-Verbindung 0,1 µg/kg/Tag bis 1 µg/kg/Tag beträgt.

13. Verwendung nach einem der Ansprüche 1-12, wobei die Dosierung des Aldosereduktasehemmers 0,01 mg/Tag bis 10 mg/Tag, vorzugsweise 0,1 mg/Tag bis 3 mg/Tag, besonders bevorzugt weniger als 2 mg/Tag beträgt.

## Revendications

1. Utilisation d'un composé GLP-1 et d'un inhibiteur d'aldose réductase pour la préparation d'un ou plusieurs médicaments destinés au traitement d'une neuropathie diabétique chez un patient en ayant besoin.

2. Utilisation selon la revendication 1, dans laquelle le composé GLP-1 est un dérivé stable d'un analogue de GLP-1.

3. Utilisation selon l'une quelconque des revendications 1 et 2, dans laquelle le composé GLP-1 est Arg³⁴, Lys²⁶(N^{ε}- (γ-Glu (N^{α}-hexadécanoyl)))-GLP-1 (7-37).

4. Utilisation selon la revendication 1, dans laquelle le composé GLP-1 est l'exendine-4 ou un analogue ou dérivé de celle-ci.

5. Utilisation selon la revendication 1, dans laquelle l'inhibiteur d'aldose réductase est le Fidarest.

6. Utilisation selon l'une quelconque des revendications 1 à 5, dans lequel le composé GLP-1 est administré selon un protocole qui comprend de plus l'administration d'un inhibiteur d'aldose réductase.

7. Utilisation selon l'une quelconque des revendications 1 à 6, dans laquelle le composé GLP-1 et l'inhibiteur d'aldose réductase sont administrés conjointement.

8. Utilisation selon l'une quelconque des revendications 1 à 7, dans laquelle le composé GLP-1 est un médicament à usage parentéral.

9. Utilisation selon l'une quelconque des revendications 1 à 8, dans laquelle l'inhibiteur d'aldose réductase est un médicament à usage oral.

10. Utilisation selon l'une quelconque des revendications 1 à 9, dans laquelle le composé GLP-1 et l'inhibiteur d'aldose réductase sont administrés à des doses sub-optimales.

11. Utilisation selon l'une quelconque des revendications 1 à 10, dans laquelle la dose de composé GLP-1 va de 0,5 µg/kg/jour à 10 µg/kg/jour.

12. Utilisation selon l'une quelconque des revendications 1 à 11, dans laquelle la dose de composé GLP-1 va de 0,1 µg/kg/jour à 1 µg/kg/jour.

13. Utilisation selon l'une quelconque des revendications 1 à 12, dans laquelle la dose de l'inhibiteur d'aldose réductase va de 0,01 mg/jour à 10 mg/jour, de préférence de 0,1 mg/jour à 3 mg/jour, et tout spécialement est inférieure à 2 mg/jour.
